# EUROPÄISCHE PATENTSCHRIFT

(11) **EP 0 668 079 B1**
(45) Veröffentlichungstag und Bekanntmachung des Hinweises auf die Patenterteilung: **23.04.2003**
(21) Anmeldenummer: 95100284.9
(22) Anmeldetag: 11.01.1995
(51) Int. Cl.: A61L 15/32, A61L 31/00, A61F 13/06, A61F 13/10

(54) **Wundabdeckungsmaterial auf Basis von Kollagenfasern in anatomisch angepasster Form**
Anatomically adapted wound covering material based on collagen fibres
Matériau de recouvrement des plates à base de fibres de collagène anatomiquement adapté

(30) Priorität: 19.02.1994 DE 4405130
(43) Veröffentlichungstag der Anmeldung: 23.08.1995
(73) Patentinhaber: Naturin GmbH & Co, D-69469 Weinheim (DE)
(72) Erfinder: Kirk, Stephen John, D-69469 Weinheim (DE); Maser, Franz Dr., D-68159 Mannheim (DE); Peiffer, Bernd, D-69234 Dielheim (DE)
(74) Vertreter: Siewers, Gescha, Dr.

(56) Entgegenhaltungen:
- EP-A- 0 331 786
- DE-A- 2 734 503
- DE-A- 2 943 520
- DE-A- 4 227 681

## Beschreibung

Die Erfindung betrifft eine Wundabdeckungsmaterial auf Basis von Kollagenfasern in anatomisch angepaßter Form.

In der deutschen Patentanmeldung DE 42 27 681 wird ein Wundabdeckungsmaterial auf Basis von Kollagenfasern beschrieben, das sich gegenüber den vorbekannten Kollagenfolien durch besondere Elastizität und Adhärenz auszeichnet und das praktisch keine allergisierende Wirkung aufweist. Ein besonderer Vorzug dieser Folie liegt darin, daß das Ausgangsmaterial in großen Mengen hergestellt werden kann und daß die Produktionskosten der Folie im Vergleich zu den bisher bekannten Verfahren relativ niedrig sind. Bisher wird diese Folie zur Abdeckung von Wunden, insbesondere Brandwunden verwendet.

Problematisch sind Wundabdeckungen allerdings immer dann, wenn die Wunde oder die Hautverletzung sich an den bewegten Extremitäten eines Menschen oder Tieres befindet, denn durch die Bewegung wird eine zielgerichtete Haftung von Verbänden deutlich erschwert. Die entsprechenden Körperteile müßten mehr oder weniger stark ruhiggestellt werden, um ein Verrutschen des aufgelegten Verbandes oder der Abdeckung zu verhindern. Die Ruhigstellung aber kann bei längerer Dauer die Beweglichkeit der Gelenke beeinträchtigen und ist für den Patienten subjektiv unangenehm. Man hat daher schon seit längerem in der Human- und Veterinärmedizin versucht, diese Problemzonen dadurch zu versorgen, daß man über Hand bzw. Arm oder Fuß bzw. Bein das Abdeckungsmaterial in Form von Handschuhen bzw. Schuhen oder Stiefeln zieht. Hierbei handelt es sich häufig um Textilhandschuhe bzw.-schuhe, die ggf. innen oder außen mit einer Kunststoffbeschichtung versehen sind und die über das geschädigte Körperteil übergestülpt werden. Der Handschuh bzw. Schuh dient zum einen als Schutz der verletzten Haut, kann andererseits aber auch als Trägermaterial von Arzneimittelwirkstoffen wie beispielsweise Salben o.ä. eingesetzt werden. Diese Handschuhe bzw. Schuhe oder Stiefel haben den Vorteil der körpergerechten Formgebung und der geforderten Wasserdampfdurchlässigkeit, während Hauthaftungsprobleme beim Verrutschen der Abdeckung wegfallen. Allerdings weisen die bisher üblichen Handschuhe bzw. Schuhe auch deutliche Nachteile auf, so die relativ hohen Kosten durch den täglich notwendigen Wechsel, die Notwendigkeit der Entsorgung als Sondermüll und außerdem in medizinischer Sicht häufig einen Wärme- und Feuchtigkeitsstau im Inneren, da die Gas- und Wasserdurchlässigkeit zumindest bei beschichteten Geweben bei stark sezernierenden Verletzungen häufig nicht ausreicht.

Man hat daher auch schon sogenannte "flüssige Handschuhe" vorgeschlagen, also die Verwendung ganz dünner direkt auf die Haut aufgesprühter Filme bestimmter physiologisch unbedenklicher Polymere wie beispielsweise von Polylactiden, die erst nach einer gewissen Zeit vom Körper hydrolisiert und abgebaut werden und bis dahin eine verletzte Haut abdecken bzw. schützen. Zwar sind diese aufgesprühten Filme durchaus geeignet, die Haut bis zu einem gewissen Grade vor chemischen oder mikrobiologischen Angriffen zu schützen und die Wundheilung zu erleichtern, aber sie bieten keinerlei mechanischen Schutz, was ein wesentlicher Nachteil dieser Versorgung ist. Außerdem können diese Flüssigverbände nur bei kleineren und oberflächlichen Wunden eingesetzt werden. Hinzu kommt, daß sie Lösungsmittel enthalten und daß beim Aufsprühen daher unterschiedliche Dicken entstehen können. Es besteht daher immer noch ein Bedürfnis nach Wundabdeckungsmaterialien für menschliche oder tierische Extremitäten, die die geschilderten Nachteile nicht aufweisen.

Erfindungsgemäß wird nunmehr ein Wundabdeckungsmaterial auf Basis von Kollagenfasern vorgeschlagen, das im wesentlichen aus unlöslichem teilmodiziertem Kollagen mit folgenden Parametern besteht, nämlich Amidstickstoff 0,18 mmol/g bis 0,40 mmol/g, Glucosamin- und Galactosamingehalt je kleiner als 5 µmol/g, Schrumpfungstemperatur 45°C bis 60 °C und isoelektrischer Punkt von pH 4,3 bis 6,0, und das dadurch gekennzeichnet ist, daß es als geformte Verbände in der Form geschlossener Handschuhe, Schuhe, Stiefel oder als Pfotenumhüllung mit einer Öffnung zum Anziehen vorliegt.

Vorzugsweise ist das Material als Handschuh mit oder ohne Stulpe, falls auch noch Armverletzungen mitzuversorgen sind, oder als Schuh oder Stiefel entsprechend den Konturen der menschlichen oder tierischen Extremitäten ausgebildet. Diese im folgenden zusammenfassend als geformte Verbände bezeichneten Materialien können bei zahlreichen Verletzungen der Haut stationär oder ambulant eingesetzt werden, beispielsweise bei Verbrennungen, bei Hautverlusten aufgrund äußerer Einwirkungen wie Schürfwunden oder bei Hauterkrankungen infektiöser oder genetischer Art. Diese geformten Verbände können aber auch bei Erkrankungen oder Verlust der Nägel oder Krallen bis zur Ausheilung verwendet werden.

Erfindungsgemäß werden die aus der Patentanmeldung DE 42 27 681.0) bekannten Flachfolien zur Herstellung der geformten Verbände eingesetzt, indem zwei derartige Folien übereinander gelegt und in der gewünschten Form an deren Rändern verschweißt oder verklebt und dann aus der Folie ausgestanzt werden. Das Verschweißen von Kollagenfolien in Formwerkzeugen oder das Verkleben unter Einsatz eines geeigneten Klebers sind Stand der Technik. In einer bevorzugten Ausführungsform ist vorgesehen, daß die geformten Verbände, insbesondere auch für den Einsatz in der Pädiatrie oder Veterinärmedizin an der Öffnung hautseitig mit einem Kleberand aus einem physiologisch unbedenklichen haut- bzw. fellhaftenden Kleber versehen sind. Derartige hauthaftende Klebstoffe sind bekannt, wobei vorzugsweise solche auf Polylactidcopolymerbasis eingesetzt werden, die restlos zu körpereigenen Stoffen abbaubar sind. Diese Kleberänder werden nach dem Ausstanzen aufgetragen und mit einer Trennfolie abgedeckt, um ein vorzeitiges Verkleben zu verhindern. Falls gewünscht, können die geformten Verbände auch gleichzeitig als Trägermaterial für Pharmazeutika eingesetzt werden, wenn beispielsweise Wunden gleichmäßig mit Antibiotika versorgt werden sollen. Arzneistoffe können entweder schon bei der Herstellung der Folien eingearbeitet werden oder sie werden bei der Herstellung der geformten Verbände zwischen die zwei zur Herstellung dienenden Folien eingebracht, wodurch eine ganz besonders gleichmäßige Verteilung ermöglicht wird.

Die erfindungsgemäßen geformten Verbände in Form von Handschuhen, Schuhen oder Stiefeln bzw. Pfotenverbänden verfügen über besonders angenehme Trageeigenschaften, da die Bewegung des Trägers im Alltag nur sehr geringen oder keinen Einschränkungen unterliegt. Sie weisen eine hohe Affinität zur Haut auf bei hoher Wasserdampfdurchlässigkeit und guter Luftpermeabilität. Aufgrund des verwendeten Materials ist eine Allergesierungsgefahr praktisch nicht vorhanden. Die Handhabung ist einfach und sauber. Wichtig ist insbesondere aber auch die Tatsache, daß gegenüber den bisher üblichen Textilprodukten längere Tragezeiten möglich sind, so daß ein Wechsel nicht täglich erfolgen muß. Schließlich kommt hinzu, daß die Produkte, da voll biologisch abbaubar, nur eine geringe Umweltbelastung darstellen und darüber hinaus im Vergleich zu den vorbekannten Produkten eine kostengünstige Herstellung ermöglichen.

Die Weiterentwicklung und Verbesserung des Wundabdeckungsmaterials wird anhand der Beispiele näher erläutert:

### Beispiel 1

Zwei nach Beispiel 1 des Hauptpatentes hergestellte Kollagenflachfolien werden aufeinander gelegt und in einem Siegelwerkzeug verschweißt. Das Siegelwerkzeug hat die vorgegebene Form beispielsweise eines Handschuhs, eines Schuhs oder einer Umhüllung für eine Hunde- oder Katzenpfote. Durch geeignete Temperaturwahl und Druckvorgabe werden die beiden Folien durch die Backen in der gewünschten Form an deren Rändern verschweißt; der geformte Verband wird dann an den Schweißnähten entlang ausgestanzt.

### Beispiel 2

Zwei aufeinandergelegte Kollagenfolien wie in Beispiel 1 werden durch ein Formwerkzeug unter Zuhilfenahme eines geeigneten Klebers auf Gelatinebasis in der Form eines Handschuhes verklebt. Der Handschuh wird anschließend entlang der Klebenaht ausgestanzt.

Werden Handschuhe mit Stulpen hergestellt, so kann daran anschließend die Stulpe teilweise umgeschlagen und am Rand mit einem hauthaftenden Kleberand versehen werden, der bis zum Gebrauch mit einer Trennfolie abgedeckt wird.

## Patentansprüche

1. Wundabdeckungsmaterial auf Basis von Kollagenfasern, das im wesentlichen aus unlöslichem teilmodifiziertem Kollagen mit folgenden Parametern besteht, nämlich Amidstickstoff 0,18 mmol/g bis 0,40 mmol/g, Glucosamin- und Glactosamingehalt je kleiner als 5 µmol/g, Schrumpfungstemperatur 45°C bis 60°C und isoelektrischer Punkt von pH 4,3 bis 6,0, **dadurch gekennzeichnet, daß** es als geformte Verbände in der Form geschlossener Handschuhe, Schuhe, Stiefel oder als Pfotenumhüllung mit einer Öffnung zum Anziehen vorliegt.

2. Wundabdeckungsmaterial nach Anspruch 1, **dadurch gekennzeichnet, daß** die Nähte verschweißt oder verklebt sind.

3. Wundabdeckungsmaterial nach Anspruch 1 oder 2, **dadurch gekennzeichnet, daß** die Öffnung hautseitig mit einem Kleberand versehen ist.

## Claims

1. A wound covering material based on collagen fibres, substantially made up of insoluble part-modified collagen with the following parameters, namely amide nitrogen 0.18 mmol/g to 0.40 mmol/g, glucosamine and glactosamine content each lower than 5 µmol/g, shrinkage temperature 45°C to 60°C and an isoelectric point of pH 4.3 to 6.0, **characterised in that** it is available as shaped dressings in the form of closed gloves, shoes, boots or as a paw casing with an opening for putting it on.

2. A wound covering material according to Claim 1, **characterised in that** the seams are welded or glued together.

3. A wound covering material according to Claim 1 or 2, **characterised in that** the opening is provided with an adhesive edge at the side thereof facing the skin.

## Revendications

1. Matériau pour couvrir des plaies à base de fibres de collagène, formé essentiellement de collagène non soluble, partiellement modifié, avec les paramètres suivants: à savoir azote amidé 0,18 mmol/g à 0,40 mmol/g, teneur en glucosamine et en galactosamine inférieure à 5 µmol/g chaque, température de retrait 45°C à 60°C, point isoélectrique de pH 4,3 à 6,0, **caractérisé en ce qu'**il se présente sous la forme de pansements moulés, tel que des gants, des chaussures, des bottes fermés ou d'enveloppes pour les membres avec une ouverture pour les enfiler.

2. Matériau pour couvrir des plaies selon la revendication 1, **caractérisé en ce que** les coutures sont soudées ou collées.

3. Matériau pour couvrir des plaies selon la revendication 1 ou 2, **caractérisé en ce que** l'ouverture, côté peau est pourvue d'un ruban adhésif.
